Europäisches Patentamt

European Patent Office

Office européen des brevets

(19)

(11) Numéro de publication : **0 428 446 A1**

(12) **DEMANDE DE BREVET EUROPEEN**

(21) Numéro de dépôt : **90403203.4**

(22) Date de dépôt : **12.11.90**

(51) Int. Cl.⁵ : **A61B 1/00, A61B 17/22**

(30) Priorité : **14.11.89 FR 8914904**

(43) Date de publication de la demande :
**22.05.91 Bulletin 91/21**

(84) Etats contractants désignés :
**CH DE GB IT LI**

(71) Demandeur : **Croisy, Renaud**
**15 rue Marignac**
**CH-1206 Geneve (CH)**
Demandeur : **Croisy, Nicolas**
**15 rue Marignac**
**CH-1206 Geneve (CH)**

(72) Inventeur : **Croisy, Renaud**
**15 rue Maignac**
**CH-120 Genève (CH)**
Inventeur : **Croisy, Nicolas**
**15 rue Maignac**
**CH-120 Genève (CH)**

(74) Mandataire : **Mongrédien, André et al**
**c/o SOCIETE DE PROTECTION DES**
**INVENTIONS 25, rue de Ponthieu**
**F-75008 Paris (FR)**

(54) Endoscope simplifié d'observation et d'intervention par tirs lasers dans une cavité du corps humain.

(57)    Endoscope simplifié d'observation et d'intervention par tirs Laser dans une cavité du corps humain (22) depuis L'extérieur de celui-ci, caractérisé en ce qu'il est composé d'une multifibre optique unique (4) entourée d'une gaine plastique (6), les fibres élementaires étant réparties en deux groupes, dont le premier (10) transmet simultanément depuis L'extérieur vers la zone à traiter la lumière d'éclairage et la lumière d'un Laser de traitement et dont le second (12) transmet, en sens inverse depuis la zone à traiter vers l'extérieur, la lumière d'observation réfléchie par cette zone à traiter.

FIG. 1

FIG. 2

EP 0 428 446 A1

## ENDOSCOPE SIMPLIFIE D'OBSERVATION ET D'INTERVENTION PAR TIRS LASERS DANS UNE CAVITE DU CORPS HUMAIN

La présente invention se rapporte d'une façon générale au domaine médical de l'observation et du traitement du corps humain à l'aide de sondes endoscopiques que l'on introduit à travers un conduit interne naturel ou artificiel de ce corps.

On connaît déjà des sondes ou endoscopes permettant d'effectuer des observations et/ou certains traitements dans des zones déterminées d'une cavité du corps humain. De telles sondes sont utilisées couramment, par exemple en urologie et en gastroentérologie. Dans leur constitution actuelle la plus générale, elles comportent principalement dans une gaine cylindrique en matière synthétique ou plastique pleine, servant de cathéter et s'étendant entre une tête d'observation et de commande externe manoeuvrée par le chirurgien et une extrémité d'intervention interne, un certain nombre de conduits creux longitudinaux destinés à recevoir des moyens d'éclairage, des moyens d'observation et des moyens de traitement.

L'apparition depuis quelques années des fibres optiques a conduit à perfectionner de façon notable ces appareils en permettant l'éclairement et l'observation plus facile de la zone de travail ; par ailleurs, des sondes laser constituées d'une fibre optique transmettant le rayonnement d'un laser de puissance sont également très couramment utilisées pour obtenir, selon la longueur d'onde de la lumière laser, soit un brûlage local d'une partie du corps indésirable, soit la cassure d'un calcul néphrétique. Des sondes d'intervention endoscopiques de ce genre sont décrites par exemple dans les documents GB-A-2 167 668 publié le 4 juin 1968 et WO 89/00023, publié le 12 janvier 1989.

Dans la demande internationale WO 89/00023 du 12 janvier 1989, on a cherché, par une structure particulière, à résoudre notamment le problème de la miniaturisation de tels endoscopes, problème dont il est facile de mesurer toute l'importance dès lors qu'il s'agit d'instruments destinés à être introduits dans les cavités du corps humain où ils sont plus ou moins bien tolérés ou acceptés.

Quoi qu'il en soit, l'état de miniaturisation obtenu à l'aide de la structure endoscopique de ce même document, reste sous la dépendance des dimensions diamétrales de la gaine en plastique ou cathéter qui ne pouvait pratiquement pas descendre en dessous de 2 mm de diamètre. Cette dimension minimale atteinte était en partie la conséquence de la nécessité de permettre à la gaine de servir de logement à l'ensemble des moyens d'éclairage, d'observation, de traitement et d'irrigation du site à traiter.

Or, les demandeurs ont trouvé qu'à l'aide de moyens simples à mettre en oeuvre et consistant essentiellement d'une part à supprimer la gaine pleine en matière plastique et d'autre part à véhiculer par la même fibre la lumière d'éclairage et la lumière laser de traitement, il était possible de réduire encore de façon très importante le diamètre d'un tel endoscope.

La présente invention a pour objet un endoscope simplifié d'observation et d'intervention par tirs laser dans une cavité du corps humain depuis l'extérieur de celui-ci, caractérisé en ce qu'il est composé d'une multifibre optique unique entourée d'une gaine plastique, les fibres élémentaires étant réparties en deux groupes, dont le premier transmet simultanément depuis l'extérieur vers la zone à traiter la lumière d'éclairage et la lumière d'un laser de traitement et dont le second transmet, en sens inverse depuis la zone à traiter vers l'extérieur, la lumière d'observation réfléchie par cette zone à traiter.

Comme on le comprend de ce qui précède, on a supprimé complètement la gaine plastique formant le cathéter et dans des canaux de laquelle était logées les différentes fibres optiques nécessaires aux instruments de l'art antérieur au profit d'une multifibre optique entourée d'une gaine plastique qui constitue à elle seule l'ensemble du cathéter introduit dans le corps humain. On parvient ainsi, conformément à l'invention, au stade ultime de la miniaturisation possible des endoscopes d'intervention.

Pour assurer l'ensemble des fonctions minimales d'observation et de traitement qui rendent opérationnel un tel endoscope, on définit dans la multifibre unique deux groupes de fibres élémentaires distinctes dont chacun a sa fonction propre. Le premier groupe transmet simultanément la lumière d'éclairage et la lumière du laser de traitement depuis l'extérieur jusqu'au lieu d'intervention et le deuxième groupe transmet en retour et par conséquent en sens inverse de la lumière d'observation réfléchie par la zone à traiter.

La séparation dans la multifibre des deux groupes de fibres élémentaires précédents qui conduisent à réaliser une face d'entrée et une face de sortie distinctes, peut être réalisée selon l'invention de deux manières différentes.

Dans un premier mode de mise en oeuvre, les deux groupes de fibres élémentaires sont séparés par une découpe partielle latérale de l'extrémité extérieure de la multifibre délimitant dans celle-ci une face d'entrée et une face de sortie, spatialement distinctes.

La face d'entrée reçoit la lumière d'éclairage qui peut être soit une lumière ordinaire, soit la lumière d'un laser de couleur blanche par exemple, ainsi que la lumière du laser énergétique que l'on superpose à la précédente pendant les faces d'intervention proprement dites.

Le second groupe de fibres élémentaires débouche à l'extérieur sur la face de sortie utilisée de façon classique par un appareil de vision qui permet de voir le champ d'intervention.

Selon un second mode de mise en oeuvre de l'invention, les deux groupes de fibres élémentaires sont identifiés directement par éclairement à l'aide d'une tache de lumière laser sensiblement circulaire, projetée sur une section de la multifibre, dont la surface concerne un nombre restreint de monofibres, et qui constitue la face d'entrée de la multifibre.

Dans ce second mode de mise en oeuvre, les faces d'entrée et de sortie de la lumière sont dans un même plan de section droite de la multifibre et la zone d'entrée, qui correspond à une petite tache lumineuse circulaire émise nécessairement par un rayon laser pour parvenir à une définition suffisamment précise, ne concerne qu'un certain nombre par exemple le tiers, des monofibres élémentaires constituant l'ensemble de la multifibre optique.

Comme dans le mode de réalisation précédent, la face d'entrée de la multifibre qui véhicule simultanément les deux lumières laser d'éclairage et de traitement est utilisée en permanence par le laser d'eclairage et de façon partielle et temporaire par les impulsions du tir du laser d'intervention.

De toute façon l'invention sera mieux comprise en se référant aux figures schématiques 1 et 2 qui décrivent un endoscope simplifié conforme à l'invention et sur lesquelles :

— la figure 1 montre le premier mode de séparation des deux groupes de fibres élémentaires et

— la figure 2, le deuxième mode de séparation de ces mêmes deux groupes de fibres élémentaires.

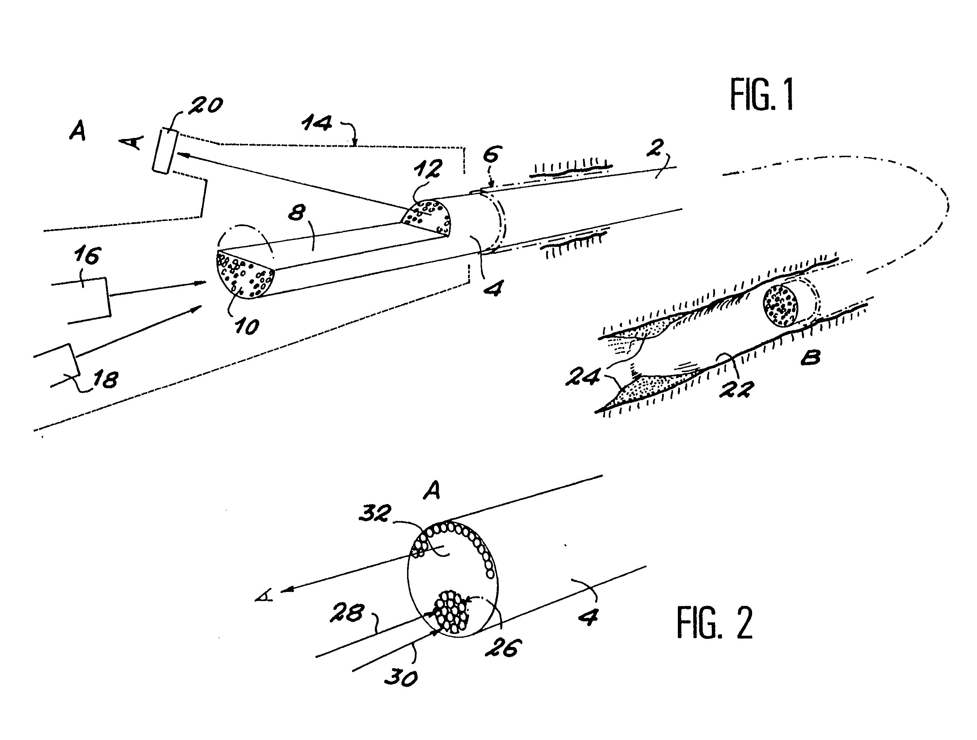

Dans la figure 1, on a représenté très schématiquement le cathéter 2 d'un système endoscopique constitué d'une multifibre optique unique 4 entourée de sa gaine de plastique 6 et dont simplement les deux extrémités d'entrée A et de traitement B ont été dessinées.

Selon l'invention, à l'extrémité A, la multifibre optique 4 est découpée selon une découpe partielle 8 permettant de définir dans les fibres élémentaires, un premier groupe 10 constituant la zone d'entrée et un deuxième groupe 12 constituant la zone de sortie de la lumière. A l'extrémité A, une tête d'observation et de commande 14 comporte un laser énergétique 16 conduisant sur la face d'entrée 6, l'énergie lumineuse servant au traitement de la zone d'intervention et une source d'éclairage 18 qui peut être soit une source de lumière normale, soit si l'on préfère, un laser d'éclairage sur la même face d'entrée 10.

La face de sortie 12 renvoie la lumière réfléchie sur la zone de traitement B vers un instrument de vision, du genre lunettess 20, permettant à l'observateur de regarder la situation de la zone de traitement.

A l'extrémité B ou extrémité de traitement, le cathéter 2 se termine dans un conduit du corps humain 22, par exemple ici un vaisseau sanguin sur les parois duquel se trouvent des athéromes 24 qu'il s'agit de détruire à l'aide de la lumière du laser de traitement 16.

Selon l'invention, la fibre optique 4 est donc divisée en deux zones, l'une d'éclairage et d'intervention et l'autre de vision, dans lesquelles la lumière se propage simultanément en sens inverse.

La figure 2 représente un autre mode de mise de l'invention dans lequel les deux faces d'entrée et de sortie du côté de la zone de commande A de la multifibre optique unique 4, sont séparées simplement par le fait qu'une surface 26 définie par l'impact d'un faisceau laser d'éclairage 28 sur la face d'entrée définit une tache ou une zone ne concernant qu'une partie des fibres élémentaires de la monofibre 4. Cette surface circulaire 26 sert donc d'entrée à la fois pour la lumière laser d'éclairage 28 et la lumière laser de traitement 30. Selon l'invention, l'homme de métier saura choisir la surface de la zone d'impact lumineuse 26, c'est-à-dire en fait, la partie du nombre des fibres élémentaires de la multifibre 4 réservée à la transmission de lumière vers la zone d'intervention par rapport au nombre des fibres élémentaires constituant la surface complémentaire 32 réservée à la transmission en sens inverse de la lumière d'observation en provenance de la zone d'intervention B vers l'oeil de l'observateur. Cette proportion peut varier dans des limites importantes ; couramment toutefois on peut admettre que la zone 26 d'entrée de la multifibre concerne à peu près le tiers des 2500 fibres élémentaires que comporte ainsi la multifibre 4. La lumière du laser d'intervention peut avoir également, selon les cas spécifiques de traitements envisagés, une puissance variant par exemple de 1 à 10 watts.

L'endoscope ainsi réalisé comporte une multifibre 4 d'un diamètre de 0,35 mm entourée d'une gaine de polyamide de 0,05 mm, ce qui conduit à un cathéter dont les dimensions diamétrales sont de l'ordre de 0,40 à 0,45 mm. On peut ainsi mesurer de façon concrète, le progrès réalisé par rapport aux endoscopes de l'art antérieur dont le diamètre ne pouvait guère descendre en dessous de 2 mm.

## Revendications

1. Endoscope simplifié d'observation et d'intervention par tirs laser dans une cavité du corps humain (22) depuis l'extérieur de celui-ci, caractérisé en ce qu'il est composé d'une multifibre optique unique (4) entourée d'une gaine plastique (6), les fibres élémentaires étant réparties en deux groupes, dont le premier (10) transmet simultanément depuis l'extérieur vers la zone à traiter la lumière d'éclairage et la lumière d'un laser de traitement et dont le second (12) transmet, en sens inverse depuis la zone à traiter vers l'extérieur, la lumière d'observation réfléchie par cette zone à traiter.

2. Endoscope selon la revendication 1, caractérisé en ce que les deux groupes de fibres élémentaires sont séparés par une découpe partielle latérale (8) de l'entrée de la multifibre (4), délimitant dans celle-ci une face d'entrée et une face de sortie spatialement distinctes.

3. Endoscope selon la revendication 1, caractérisé en ce que les deux groupes de fibres élementaires sont identifiées directement par l'éclairement d'une tache de lumière laser sensiblement circulaire (28), projetée sur une section de la multifibre (4), dont la surface concerne un nombre restreint de monofibres, et qui constitue la face d'entrée de la multifibre.

FIG. 1

FIG. 2

EP 0 428 446 A1

Office européen
des brevets

**RAPPORT DE RECHERCHE EUROPEENNE**

Numero de la demande

EP    90 40 3203

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | US-A-4830460 (T.GOLDENBERG)<br>* colonne 10, lignes 23 - 34 *<br>* colonne 11, ligne 45 - colonne 13, ligne 25; figures 6-12 * | 1 | A61B1/00<br>A61B17/22 |
| Y | | 2, 3 | |
| | --- | | |
| Y | US-A-3329074 (N.R.GOSSELIN)<br>* colonne 7, ligne 69 - colonne 8, ligne 64; figures 18-21 * | 2 | |
| | --- | | |
| Y | EP-A-0297190 (MCM LABORATORIES INC.)<br>* abrégé; figures 4-7 * | 3 | |
| | --- | | |
| A | EP-A-0326400 (A.PRITCHARD)<br>* abrégé; figures 4a-4e * | 1 | |
| | --- | | |
| A | US-A-4272156 (K.ISHIBASHI ET AL.)<br>* le document en entier * | 1, 2 | |
| | ----- | | |
| | | | **DOMAINES TECHNIQUES RECHERCHES (Int. Cl.5 )**<br><br>A61B<br>A61F<br>G02B |

Le présent rapport a été établi pour toutes les revendications

| Lieu de la recherche | Date d'achèvement de la recherche | Examinateur |
|---|---|---|
| LA HAYE | 18 FEVRIER 1991 | HUNT B.W. |

EPO FORM 1503 03.82 (P0402)

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
......................................................................................
& : membre de la même famille, document correspondant